## Europäisches Patentamt

## European Patent Office

## Office européen des brevets

(11) Numéro de publication: **0 031 527 B1**

(12) **FASCICULE DE BREVET EUROPEEN**

(45) Date de publication du fascicule du brevet:
**18.04.84**

(21) Numéro de dépôt: **80107856.9**

(22) Date de dépôt: **12.12.80**

(51) Int. Cl.³: **C 12 P 21/02,** C 07 C 103/52, C 07 C 149/247

(54) **Procédé de préparation d'un oligomère de L-acide aminé par voie enzymatique, et utilisations de celui-ci.**

(30) Priorité: **28.12.79 CH 11505/79**

(43) Date de publication de la demande:
**08.07.81 Bulletin 81/27**

(45) Mention de la délivrance du brevet:
**18.04.84 Bulletin 84/16**

(84) Etats contractants désignés:
**CH DE FR LI SE**

(56) Documents cités:
**DE - A - 2 909 854**
**FR - A - 2 001 874**
**FR - A - 2 203 598**
**US - A - 4 086 136**

**AGRICULTURAL AND BIOLOGICAL CHEMISTRY Vol. 43, no. 5, Mai 1979, pages 1069-1074, Tokyo JP S. ARAI et al.: "A novel one-step process for enzymatic incorporation of amino acids into proteins: papain-catalyzed polymerization of 1-methionine ethyl ester and its regulation by adding a protein substrate"**
**HELVETICA CHIMICA ACTA, voi. 62, no. 2, 7 Mars 1979, pages 488-494, Basel CH G. ANDERSON et al.: "Papain-induced oligomerization of alpha amino-acid esters"**
**HELVETICA CHIMICA ACTA, voi. 63, no. 2, 5 Mars 1980,**

(73) Titulaire: **SOCIETE DES PRODUITS NESTLE S.A., Case postale 353, CH-1800 Vevey (CH)**

(72) Inventeur: **Jost, Rolf, Rue gambetta 40, CH-1815 Clarens (CH)**

(56) Documents cités: (suite)
**pages 375-384, Basel CH R. JOST et al.: "Papain catalyzed oligomerization of alpha aminoacids synthesis and characterization of water insoluble oligomers of 1-methionine"**
**HELVETICA CHIMICA ACTA, voi. 33, no. 3, 2 Mai 1950, pages 568-591, Basel CH M. BRENNER et al.: "Eine neue enzymatische peptidsynthese"**
**JOURNAL OF AGRICULTURAL AND TOOL CHEMISTRY, vol. 27, no. 1, janvier-février 1979, page 52-56, Washington DC US M. YAMASHITA et al.: "A one-step process for incorporation of 1-methionine into soy protein by treatment with papain"**

### Procédé de préparation d'un oligomère de L-acide aminé par voie enzymatique, et utilisations de celui-ci

La présente invention concerne un procédé de préparation d'un oligomère de L-acide aminé contenant des résidus de L-méthionine obtenu par voie enzymatique constitué essentiellement d'oligomères ayant une longueur de chaîne comprise entre 5 et 10 résidus avec une longueur moyenne de 8, ainsi que l'utilisation de celui-ci, soit comme substitut de L-méthionine dans la fortification des diètes alimentaires, soit comme produit intermédiaire dans l'obtention de L-méthionine à partir de DL-méthionine.

Certaines matières alimentaires naturellement pauvres en acides aminés soufrés, telles que les protéines de soja, les protéines de feuilles et les protéines microbiennes, peuvent ou doivent être fortifiées avec de la méthionine, dans le cadre notamment d'un usage diététique ou infantile.

La DL-méthionine, qui est produite par synthèse chimique à l'échelle de 100'000 t par an, est surtout consommée par le bétail et la volaille, car son usage en alimentation humaine est sujet à caution.

Pour l'homme, seul est actuellement envisagé l'emploi de la L-méthionine, qui est produite à partir de la DL-méthionine, dans un ordre de grandeur de 50 à 100 t par an, par séparation des deux énantiomères par voie enzymatique. De ce fait, la L-méthionine est environ 10 fois plus chère que la DL-méthionine.

Des oligomères de méthionine, principalement le dimère, ont été synthétisés par voie chimique:

— par réaction de méthionine N-protégée telle que la N-carbobenzoxyméthionine avec l'ester éthylique de la méthionine en présence d'un agent de couplage tel que le cyclohexylcarbodiimide et clivage du groupement protecteur;

— à partir des N-carboxyanhydrides ou anhydrides de Leuch's en présence d'amino-acide libre en milieu aqueux à froid à pH 10 (méthode de Hirschmann).

Ces méthodes sont coûteuses et mettent en œuvre des produits instables ou des réactifs de couplage dont la pureté doit être garantie.

On a également obtenu des poly-L-méthionines à haut poids moléculaire à partir de N-carboxyanhydrides par polymérisation en milieu anhydre mais impliquant également l'utilisation d'anhydrides de Leuch's difficiles à manipuler.

Ces méthodes n'ont cependant pas pu trouver d'application dans la préparation d'additifs alimentaires à cause de leur prix élevé et de la garantie de pureté qui est imposée par l'hygiène alimentaire.

En particulier:

— Agricultural and biological chemistry Vol. 43, No. 5, mai 79 ainsi que Journal of Agricultural and Food Chemistry Vol. 27, No. 1, janv. et fév. 1979, p. 52-56 concernent l'enrichissement de protéines de soja par greffage direct de L-méthionine catalysé par la papaïne. Bien que les auteurs aient constaté la formation d'un polymère insoluble, ils ont considéré que cette réaction secondaire était indésirable.

— Helvetica chimica acta Vol. 62, No. 2, 7.3.79, p. 488-494, concerne l'oligomérisation d'esters d'α-amino-acides catalysée par la papaïne. On notera qu'un oligomère de méthionine de degré de polymérisation 5-10, H-(Met)$_{5\text{-}10}$-OMe ne peut pas être considéré comme ayant été synthétisé car il n'a pas été caractérisé. Ce qui a été obtenu est un oligomère différent, N- protégé, Z-(Met)$_9$-OMe où Z est carbobenzoxy. D'une part la synthèse de ce dernier composé via Z-Met-OH et H-Met-OMe serait considérablement moins économique que l'oligomérisation de l'ester de méthionine seul, d'autre par le groupe protecteur Z devrait être éliminé avant toute utilisation alimentaire.

— Helvetica chimica acta, Vol. 33, No. 3, mai 1950, p. 568-591 décrit la formation de polypeptides catalysée par la chimotrypsine à partir de la méthionine. Les auteurs parlent d'un produit de réaction insoluble qui pourrait être un mélange de «polymères homologues» de peptides de méthionine sans les avoir caractérisés (seuls le di- et le tripeptide l'ayant été).

Toutes les antériorités commentées ci-dessus soit qualifient la formation d'oligomère d'acide aminé indésirable, soit n'ont pas caractérisé d'oligomère de méthionine ou utilisé une voie de synthèse beaucoup moins économique. De plus, aucune de celles-ci ne décrit ni ne suggère une méthode simple de fabrication de L-méthionine à partir du racémique DL-méthionine via l'oligomérisation. Enfin aucune des antériorités ne décrit ni ne suggère la synthèse dirigée d'hétéro-oligomères de méthionine et d'acide glutamique, asparique ou de tryptophane, thyrosine en vue d'améliorer et d'étendre les possibilités d'utilisation en supplémentation (solubilité, digestibilité).

On comprend dès lors l'intérêt de pouvoir disposer, soit d'un produit de remplacement non suspect de la L-méthionine, la libérant dans les conditions d'ingestion, soit de L-méthionine à un prix relativement modéré.

L'invention répond précisément à ces deux objectifs. Elle concerne un procédé de préparation par voie enzymatique d'un oligomère de L-acide aminé essentiellement constitué d'oligomères ayant une longueur de chaîne comprise entre 5 et 10 résidus avec une longueur moyenne de 8, dont la teneur en L-méthionine est comprise entre 60 et 90% en poids, qui est peu soluble dans l'eau et les tampons aqueux et soluble dans le diméthylsulfoxyde, a une teneur en azote total comprise entre 9,5 et 10,5% en poids et un pouvoir rotatoire spécifique $[\alpha]_D^{25}$ de —12 à —14° dans le diméthylsulfoxyde. Il est caractérisé par le fait qu'en milieu aqueux de pH compris entre 5 et 7, on fait réagir un ester de la DL-méthionine et éventuellement d'un ou de plusieurs autres aminés avec une thiol-protéase à une température où cette dernière est active, puis qu'on isole l'oligomère de L-acide aminé formé du milieu réactionnel. Selon une forme d'exécution, l'oligomère est un homo-oligomère constitué de résidus de L-méthionine.

Par l'expression «peu soluble dans l'eau», on entend indiquer que *in vitro* l'oligo-L-méthionine, donc l'homo-oligomère ne se dissout pas dans l'eau de façon sensible. Cela ne préjuge en rien, bien au contraire, de sa solubilité *in vivo* dans les milieux aqueux et les fluides biologiques.

Selon une variante, l'oligomère est un hétéro-oligomère, c'est-à-dire qu'il contient en plus des résidus méthionine, des résidus d'autres acides aminés. Un tel oligomère a une meilleure solubilité que l'homo-oligomère dans les milieux aqueux si on y introduit des résidus d'acides aminés à caractère polaire tel que l'acide glutamique ou aspartique.

En variante, il peut présenter une meilleure digestibilité vis-à-vis des enzymes pancréatiques que l'homo-oligomère de L-méthionine, par exemple par introduction de résidus d'acides aminés à caractère aromatique tel que le tryptophane, la phénylalanine ou la tyrosine, par création de sites supplémentaires de clivage pour les protéases pancréatiques, ce qui facilite l'hydrolyse enzymatique.

Par ailleurs, l'oligomère libère, dans les conditions de pH acide telles qu'on les rencontre par exemple dans l'estomac, la L-méthionine. On conçoit dès lors que, par l'intermédiaire de l'oligo-L-méthionine, il soit possible d'obtenir la L-méthionine à partir de la DL-méthionine, comme décrit plus loin dans l'exposé d'invention. Le processus global, bien que faisant appel à une intervention enzymatique, s'avère meilleur marché pour la préparation de la L-méthionine que le dédoublement enzymatique visé plus haut.

On remarque que la méthionine de départ étant la DL-méthionine, le milieu réactionnel contient la D-méthionine qui n'a pas réagi sous forme de son ester.

La préparation de l'ester de méthionine, de préférence l'ester méthylique ou éthylique, est un préalable banal, peu onéreux. Cette opération peut être réalisée d'une façon toute simple en faisant réagir la méthionine avec l'alcool correspondant en présence de chlorure de thionyle ($SOCl_2$).

Dans le cas d'un hétéro-oligomère, il suffit d'ajouter au mélange réactionnel constitué par l'ester de méthionine et la thiol-protéase le ou les acides aminés en quantité désirée, sous forme de leur ester, de préférence méthylique ou éthylique, préparé comme ci-dessus. On peut employer par exemple: les esters éthyliques de DL- ou de L-tryptophane, de DL-tyrosine, le $\alpha, \gamma$-diéthylglutamate. Si l'on part d'un acide aminé DL, on obtiendra un résidu L comme indiqué ci-dessus pour la méthionine.

Comme thiol-protéase, on utilisera de préférence la papaïne, la ficine ou la bromeline, qui sont des enzymes bon marché et abondants. On travaillera alors entre 30°C et 50°C et préférablement autour de 37°C.

Comme milieu aqueux réactionnel, on peut choisir tout simplement l'eau et on veillera alors à ce que le pH reste dans les limites ci-dessous indiquées, de préférence constant. Au fur et à mesure du déroulement de la réaction enzymatique, on ajoutera un agent alcalin, comme par exemple de la soude 2N. Une autre possibilité est d'utiliser, à titre de milieu aqueux, une solution tampon, par exemple une solution tampon de sels de sodium d'acides carboxyliques, citrate de Na, succinate de Na, acétate de Na, etc.

Le pH du milieu aqueux est déterminant pour la réaction enzymatique et doit être impérativement maintenu entre 5 et 7. Avantageusement, ce pH est choisi autour de 6,5 lorsque la réaction est conduite dans l'eau, et autour de 5,5 lorsque la réaction est conduite dans un tampon.

Lorsque la réaction enzymatique de formation de l'oligomère est terminée, le milieu réactionnel, à l'origine clair, s'est transformé en une suspension laiteuse. Avantageusement, on inactive alors l'enzyme, par exemple par un bref chauffage à 90°C, puis on sépare la phase solide qui constitue l'oligomère cherché, de la phase liquide. Outre les impuretés et les sous-produits de la réaction, le milieu réactionnel comprend en solution le D-acide aminé qui n'a pas réagi sous forme de son ester (méthylique ou éthylique).

Pour une meilleure pureté des oligomères, on lave la phase solide abondamment à l'eau. Pour faciliter la séparation des phases, on peut centrifuger.

On peut également purifier par dialyse ou par ultrafiltration, après avoir remis la phase solide en suspension dans l'eau. De préférence, on diafiltre, c'est-à-dire qu'on ultrafiltre en diluant le rétentat. On peut alors diluer de façon continue en maintenant le niveau constant, ou bien de façon discontinue, par ultrafiltration sans dilution, suivie d'un réajustement au niveau d'origine avant d'ultrafiltrer à nouveau.

On obtient ainsi l'oligomère de L-acide aminé dont les caractéristiques sont énoncées plus haut et dont les moyens de caractérisation seront indiqués dans les exemples. Mis en présence de pepsine en milieu acide, l'oligomère selon l'invention est hydrolysé et on recueille en majorité la L-méthionine, accompagné de di-L-méthionine et tri-L-méthionine. C'est dire que, dans le cas d'un homo-oligomère, l'oligo-L-méthionine peut jouer le rôle de la L-méthionine proprement dite dans la fortification des matières alimentaires pauvres en méthionine.

Dans des conditions de forte acidité, l'oligo-L-méthionine est clivée en L-méthionine, avec un taux de racémisation négligeable. La préparation de l'oligo-L-méthionine à partir de DL-méthionine, suivie de son hydrolyse en milieu acide, constitue donc un moyen élégant de préparer la L-méthionine à partir de son racémique.

Selon une première forme d'exécution du procédé selon l'invention, on dissout dans l'eau un chlorhydrate d'un ester de méthionine en concentration comprise de préférence entre 2 et 4 M. On maintient le pH constant, à 6,5, durant toute la réaction en ajoutant NaOH 2 N.

L'enzyme, à savoir la papaïne, la broméline ou la ficine, est ajoutée à raison de 2% en poids, de préférence en deux additions de 1% chacune. En fin de réaction, après environ 6 heures, une suspension laiteuse s'est formée et celle-ci est chauffée brièvement à 90°C pour inactiver l'enzyme qu'elle contient. Les deux phases solide et liquide sont séparées, puis la phase solide, qui représente l'oligo-L-méthionine est lavée par diafiltration.

Dans une deuxième forme d'exécution, la réaction est conduite à un pH autour de 5,5, dans un tampon de citrate, succinate, ou acétate de sodium.

Le même processus expérimental que ci-dessus peut être employé dans le cas d'un hétéro-oligomère en dissolvant dans l'eau dans la première étape les différents chlorhydrates d'acides aminés envisagés dans les proportions choisies.

Les exemples suivants illustrent l'invention et présentent les méthodes de caractérisation de l'oligo-L-méthionine. Dans ces exemples, les proportions et pourcentages sont exprimés en valeurs pondérales.

*Exemple 1*

On prépare de façon conventionnelle du chlorhydrate de l'ester éthylique de la DL-méthionine par action du $SOCl_2$ sur une solution de DL-méthionine dans l'éthanol.

Ce chlorhydrate est alors dissout dans l'eau, à raison de 600 mg par ml (2,8 M). La solution est placée dans un pH-stat, de façon à maintenir, durant la réaction, le pH constant à une valeur de 6,5, à l'aide de NaOH 2 N.

On ajoute alors, à raison de 1% en poids d'enzyme, une solution aqueuse 0,1 M de papaïne dans la L-cystéine. Une heure plus tard, on ajoute encore 1% de l'enzyme, comme précédemment.

Après 6 heures de réaction à 37°C, au pH maintenu rigoureusement constant à 6,5, le milieu réactionnel, initialement clair, s'est transformé en une suspension laiteuse. On chauffe alors brièvement à 90°C pour inactiver l'enzyme, puis on sépare la phase solide, qu'on rince abondamment avec de l'eau.

Cette phase solide, qui représente l'oligo-L-méthionine brute, est remise en suspension dans 20 fois son poids d'eau, puis placée dans une cellule d'ultrafiltration, munie d'une membrane ® Amicon UM 10, sous une pression d'azote de 2,5 atm et à 4°C. L'ultrafiltration est poursuivie jusqu'à une réduction de volume au 20ème, puis le volume initial est restauré par addition d'eau et le processus est répété. Quand environ 100 volumes de filtrats ont été récupérés, l'ultrafiltration est arrêtée et le rétentat est séché par lyophilisation. Ce rétentat représente l'oligo-L-méthionine purifiée.

*Caractérisation de l'oligo-L-méthionine*

I. Le spectre infra-rouge (I.R.) effectué à l'état solide à la concentration 1% dans KBr montre les bandes caractéristiques:

3230 cm$^{-1}$ (s) :   N-H stretching caractéristique d'une structure $\beta$.

1710 cm$^{-1}$ (w):   C = O: groupes carboxyles terminaux

1620 cm$^{-1}$ (s) :   C = O stretching: bande amide I

1520 cm$^{-1}$ (s) :   N = H déformation: bande amide II

700 cm$^{-1}$ (m):   vibration hors du plan: bande amide V

II. Le spectre de résonance magnétique nucléaire du proton (R.M.N.) de l'oligo-L-méthionine brute dans le diméthylsulfoxyde (DMSO-d6) à 80 MHz montre les signaux caractéristiques:

8,2 ppm :   multiplet (2): groupement amino terminal H$_2$N-

4,4 ppm :   multiplet (env. 10 × 2) × protons méthylène -O-CH$_2$-CH$_3$

2,4 ppm :   multiplet
1,8 ppm :   épaule     } protons $\alpha$, $\beta$ et $\gamma$

2,0 ppm :   singlet (env. 10 × 2): protons thioester -S -CH$_3$

1,2 ppm :   triplet (3): protons méthyl -O -CH$_2$-CH$_3$

III. Un profil d'élution sur résine ® Sephadex G25 fine dans le diméthylsulfoxyde (DMSO) montre que l'oligo-L-méthionine est relativement homogène, le pic principal correspondant, d'après les volumens d'élution et les conditions opératoires, à l'octamère.

IV. D'autres éléments de caractérisation sont donnés ci-dessous:

— insoluble dans l'eau et les tampons aqueux,
— soluble dans le diméthylsulfoxyde (DMSO),
— contenu en azote total compris entre 9,5 et 10,5%, pouvoir rotatoire spécifique $[\alpha]_D^{25}$ = —12 à —14° (DMSO),
— dichroïsme circulaire $\theta$ = 1922°, deux bandes négatives à 230 et 195 nm,
— analyse élémentaire comparée à celle des analyses élémentaires théoriques (calculées) pour les oligomères de 6 à 10 résidus méthionine après clivage du groupement ester par hydrolyse: % C = 45,22; % H = 7,62; % N = 10,58, ce qui correspond à un nombre moyen de résidus de 8,
— teneur en méthionine comprise entre 78 et 90%.

*Exemple 2*

On répète ce qui est décrit à l'exemple 1, à partir de DL-méthionine, mais en utilisant comme enzyme la ficine au lieu de la papaïne.

L'oligo-L-méthionine obtenu est semblable à celle de l'exemple 1.

*Exemple 3*

On prépare l'ester méthylique de la DL-méthionine par action de $SOCl_2$ sur une solution de DL-méthionine dans le méthanol, ester méthylique qu'on isole comme chlorhydrate.

A partir de cet ester méthylique, utilisé à la place de l'ester éthylique de l'exemple 1 en proportions correspondantes, on répète ce qui est décrit dans cet exemple.

On obtient ainsi une oligo-L-méthionine semblable à celle de l'exemple 1.

*Exemple 4*

On modifie le mode opératoire décrit à l'exemple 1 en employant, comme milieu réactionnel, un tampon aqueux constitué d'une solution 1 M de citrate de sodium, dont le pH est de 5,5. Dans ce cas, le pH reste constant durant toute la réaction et, évidemment, aucune addition de NaOH n'est nécessaire; le reste des opérations est identique à ce qui est décrit dans cet exemple 1.

On obtient ainsi une oligo-L-méthionine semblable à celle qui est décrite à l'exemple 1.

*Exemple 5*

On répète ce qui est décrit à l'exemple 1 à partir de DL-méthionine en utilisant, selon les indications de l'exemple précédent, un tampon d'acétate de sodium 1 M, dont le pH est de 5,5.

On obtient une oligo-L-méthionine semblable à celle décrite aux exemples précédents.

*Exemple 6*

On soumet l'oligo-L-méthionine décrite aux exemples précédents à une digestion enzymatique à l'aide de pepsine porcine à raison de 2% en poids. Pour ce faire, l'oligo-L-méthionine est mise en suspension, à raison de 2% en poids, à pH = 2, à 37°C pendant 24 heures. Dans ce laps de temps, 85% de l'oligo-L-méthionine est transformé en produits solubles. Ceux-ci sont essentiellement composés de L-méthionine libre, à côté de traces d'ester éthylique de la L-méthionine, ainsi que de di-L-méthionine et de tri-L-méthionine.

*Exemple 7*

On soumet 1 g d'oligo-L-méthionine décrite aux exemples 1 à 5 à l'hydrolyse acide dans environ 500 ml d'acide chlorhydrique 6N à 105-110°C sous azote pendant environ 20 heures. L'acide est ensuite éliminé par distillation sous pression réduite et l'hydrolysat repris par l'eau. On y identifie la L-méthionine chromatographiquement (couche mince) et électrophorétiquement pure avec environ 1 à 2,5% de D-méthionine. La faible quantité de D-méthionine présente peut être due à la présence de D-méthionine résiduelle dans l'oligomère ou à une racémisation due à l'hydrolyse chimique. Le pouvoir rotatoire spécifique de l'acide aminé isolé et recristallisé est $[\alpha]_D^{25} = +22$ à $+23°$ (HCl 6N). Par comparaison la L-méthionine de qualité analytique du commerce a un pouvoir rotatoire $[\alpha]_D^{25} = +23,7° \pm 0,5°$.

*Exemple 8*

On dissout dans l'eau environ 1 millimole par ml du chlorhydrate de l'ester éthylique de DL-méthionine et 0,25 millimole par ml de $\alpha$, $\gamma$-diéthylglutamate. Cette solution est placée dans un pH-stat à 40°C, le pH étant maintenu à 6,5, à l'aide de NaOH 2 N. On ajoute alors de la papaïne à raison d'1 mg/ml. Après 6 h de réaction à pH constant l'enzyme est inactivé et le précipité blanc séparé par centrifugation est traité par ultrafiltration comme indiqué à l'exemple 1. Le produit est ensuite dispersé dans une solution 0,2 N de NaOH et après 2 h sous agitation à température ambiante on obtient une solution claire. On abaisse le pH de la solution à 10 par addition d'acide chlorhydrique 6 N et on traite la solution sur colonne Sephadex® G-10 pour éliminer les sels en excés et éluer les peptides. La solution neutre obtenue est concentrée sous vide et lyophilisée. L'analyse des acides aminés montre que le produit est composé de 34-35% d'acide glutamique pour environ 65% de méthionine.

La solubilité dans l'eau à 20°C de l'hétéro-oligomère est > 200 mg/ml contre < 1 mg/ml pour l'oligo-L-méthionine.

On peut, en variant les proportions acide glutamique/méthionine dans le mélange réactionnel, obtenir différentes solubilités de l'oligomère: par exemple, l'acide glutamique représentent 8,5% de l'oligomère, la solubilité est de 5-10 mg/ml.

*Exemple 9-10*

On soumet 1 millimole/ml de chlorhydrate de l'ester éthylique de DL-méthionine et 0,25 millimole/ml de chlorhydrate de l'ester éthylique de DL-tryptophane au traitement enzymatique selon l'exemple 1 et on obtient une poudre blanche insoluble dans l'eau contenant d'après l'analyse des acides aminés, 11 ± 3,5% de L-tryptophane (exemple 9).

En procédant comme ci-dessus à partir de 0,2 millimole/ml de chlorhydrate de l'ester éthylique de L-tyrosine à la place de celui du DL-tryptophane, on obtient un hétéro-oligomère contenant 14 ± 2% de L-tyrosine (exemple 10).

L'adjonction de tryptophane ou de tyrosine dans le milieu réactionnel permet de faciliter la digestion enzymatique de l'oligomère par les protéases pancréatiques comme montré ci-après:

Une dispersion à 5% des oligomères dans l'eau est soumise à une digestion par 5 mg/ml de pancréatine dans un pH-stat à pH 8 à 40°C. A différentes périodes, des échantillons sont soumis à la réaction avec la ninhydrine pour déterminer leur teneur en azote en position $\alpha$ dans la *fraction soluble*. Les résultats sont indiqués dans le tableau ci-dessous:

| Exemple | Oligomère | % d'azote en position $\alpha$ après -h de digestion | | |
|---|---|---|---|---|
| | | 1 h | 6 h | 20 h |
| 1 | méthionine | 9 | 24 | 39 |
| 9 | méthionine/ tryptophane | 29 | 49 | 79 |
| 10 | méthionine/ tyrosine | 25 | 46 | 56 |

**Revendications**

1. Procédé de préparation par voie enzymatique d'un oligomère de L-acide aminé essentiellement constitué d'oligomères ayant une longueur de chaîne comprise entre 5 et 10 résidus avec une longueur moyenne de 8, dont la teneur en L-méthionine est comprise entre 60 et 90% en poids, qui est peu soluble dans l'eau et les tampons aqueux et soluble dans le diméthylsulfoxyde, a une teneur en azote total comprise entre 9,5 et 10,5% en poids et un pouvoir rotatoire spécifique $[\alpha]_D^{25}$ de —12 à —14° dans le diméthylsulfoxyde, caractérisé per le fait qu'en milieu aqueux de pH compris entre 5 et 7, on fait réagir un ester de la DL-méthionine et éventuellement d'un ou de plusieurs autres acides aminés avec une thiol-protéase à une température où cette dernière est active, puis qu'on isole l'oligomère de L-acide aminé formé du milieu réactionnel.

2. Procédé selon la revendication 1, dans lequel l'ester de méthionine mis en œuvre est l'ester méthylique ou éthylique de la DL-méthionine.

3. Procédé selon la revendication 1, dans lequel le ou les esters des autres acides aminés mis en œuvre sont les esters méthylique ou éthylique de l'acide aspartique, de l'acide glutamique, du L-tryptophane, du DL-tryptophane ou de la DL-tyrosine.

4. Procédé selon la revendication 1, caractérisé par le fait que la thiol-protéase est la papaïne, la ficine ou la bromeline et que le milieu aqueux réactionnel est maintenu à un pH compris entre 5 et 7 au moyen d'un pH-stat ou est une solution tampon d'un sel de sodium d'un acide carboxylique.

5. Procédé selon la revendication 4, caractérisé par le fait qu'on inactive la thiol-protéase et qu'on isole l'oligomère de L-acide aminé par ultrafiltration avec dilution du rétentat au cours de l'opération d'ultrafiltration.

6. Utilisation de l'oligomère de L-acide aminé obtenu par la mise en œuvre du procédé selon la revendication 1 pour réhausser la valeur nutritive des aliments pauvres en méthionine.

7. Procédé de préparation de L-méthionine, caractérisé per le fait qu'on fait réagir un ester de DL-méthionine avec une thiol-protéase à une température où cette dernière est active, qu'on isole l'oligomère de L-méthionine formé du milieu réactionnel puis qu'on l'hydrolyse en milieu acide.

8. Procédé selon la revendication 7, caractérisé per le fait qu'on fait réagir l'ester éthylique de DL-méthionine avec une thiol-protéase en maintenant le milieu réactionnel à un pH compris entre 5 et 7, qu'on inactive la thiol-protéase, qu'on isole l'oligomère de L-méthionine obtenu par ultrafiltration avec dilution au cours de l'opération d'ultrafiltration et qu'on l'hydrolyse par un acide fort concentré.

## Patentansprüche

1. Verfahren zur enzymatischen Herstellung eines L-Aminosäuren-Oligomers, das im wesentlichen aus Oligomeren mit einer Kettenlänge von 5 bis 10 Resten und einer mittleren Kettenlänge von 8 Resten besteht, dessen Gehalt an L-Methionin zwischen 60 und 90 Gew.-% liegt, das in Wasser und wässrigen Puffern schwachlöslich und in Dimethylsulfoxid löslich ist, das einen gesamten Stickstoffgehalt zwischen 9,5 und 10,5 Gew.-% aufweist und das in Dimethylsulfoxid eine spezifische Drehung $[\alpha]_6^{25}$ von —12 bis —14° besitzt, dadurch gekennzeichnet, dass man in einem wässrigen Medium mit einem pH zwischen 5 und 7 einen Ester von DL-Methionin und gegebenenfalls von einer oder mehreren anderen Aminosäuren mit einer Thiol-Protease bei einer Temperatur, bei der letztere aktiv ist, umsetzt und hierauf das gebildete L-Aminosäuren-Oligomer aus dem Reaktionsmedium isoliert.

2. Verfahren nach Anspruch 1, bei welchem der verwendete Methioninester der Methyl- oder Äthylester von DL-Methionin ist.

3. Verfahren nach Anspruch 1, bei welchem der oder die weiteren verwendeten Ester von anderen Aminosäuren Methyl- oder Äthylester von Asparaginsäure, Glutaminsäure, L-Tryptophan, DL-Tryptophan oder DL-Thyrosin sind.

4. Verfahren nach Anspruch 1, dadurch gekennzeichnet, dass die Thiol-Protease Papain, Ficin oder Bromelin ist und dass das wässrige Reaktionsmedium mit Hilfe eines pH-Stabilisators auf einem pH zwischen 5 und 7 gehalten wird oder eine Pufferlösung eines Natriumsalzes einer Carbonsäure ist.

5. Verfahren nach Anspruch 4, dadurch gekennzeichnet, dass man die Thiol-Protease deaktiviert und das L-Aminosäuren-Oligomer durch Ultrafiltration mit Verdünnung des Retentats während der Ultrafiltration isoliert.

6. Verwendung des durch das Verfahren von Anspruch 1 erhaltenen L-Aminosäuren-Oligomers zur Steigerung des Nährwerts von an Methionin armen Nahrungsmitteln.

7. Verfahren zur Herstellung von L-Methionin, dadurch gekennzeichnet, dass man einen Ester von DL-Methionin mit einer Thiol-Protease bei einer Temperatur, bei der letztere aktiv ist, umsetzt und hierauf das gebildete L-Methionin-Oligomer aus dem Reaktionsmedium isoliert und in saurem Medium hydrolysiert.

8. Verfahren nach Anspruch 7, dadurch gekennzeichnet, dass man den Äthylester von DL-Methionin mit einer Thiol-Protease unter Einhaltung eines pH des Reaktionsmediums zwischen 5 und 7 umsetzt, die Thiol-Protease deaktiviert und das erhaltene L-Methionin-Oligomer durch Ultrafiltration isoliert und mit einer starken konzentrierten Säure hydrolysiert.

## Claims

1. A process for enzymatically preparing an oligomer of L-amino acid consisting essentially of oligomers having a chain length of from 5 to 10 residues with an average length of 8 and having an L-methionine content amounting to from 60 to 90% by weight, which is poorly soluble in water and aqueous buffers and soluble in dimethyl sulphoxide, having a total nitrogen content amounting to from 9.5 to 10.5% by weight and having a specific rotation $[\alpha]_6^{25}$ of from —12 to —14° in dimethyl sulphoxide, characterized by the fact that it comprises reacting an ester of DL-methionine and, optionally, of one or more other amino acids, with a thiol-protease in an aqueous medium having a pH value in the range from 5 to 7 at a temperature where the thiol-protease is active, and thereafter isolating the L-amino acid oligomer from the reaction medium.

2. A process as claimed in claim 1, wherein the ester is the methyl or ethyl ester of DL-methionine.

3. A process as claimed in claim 1, wherein the ester(s) of the other amino acid(s) is/are the methyl or ethyl ester(s) of aspartic acid, glutamic acid, L-tryptophane, LD-tryptophane or DL-tyrosine.

4. A process as claimed in claim 1, wherein the thiol-protease is papain, ficin or bromelin and the aqueous reaction medium is kept at a pH of from 5 to 7 by means of a pH-stat or its a buffer solution of a sodium salt of a carboxylic acid.

5. A process as claimed in claim 1, wherein the thiol-protease is inactivated and the L-amino acid oligomer isolated by ultrafiltration with dilution of the retentate during the ultrafiltration process.

6. The use of an oligomer of L-amino acid when prepared by a process as claimed in claim 1 to enhance the nutritive value of a food poor in methionine.

7. A process for preparing L-methionine which comprises reacting an ester of DL-methionine with a thiol-protease at a temperature where the thiol-protease is active, isolating the L-amino acid oligomer from the reaction medium and hydrolysing it in an acid medium.

8. A process as claimed in claim 7, which comprises reacting the ethyl ester of DL-methionine with a thiol-protease while maintaining the pH of the reaction medium at a value in the range from 5 to 7, inactivating the thiol-protease, isolating the L-amino acid oligomer by ultrafiltration with dilution of the retentate and hydrolysing it in a concentrated strong acid medium.